# EUROPÄISCHE PATENTSCHRIFT

(11) **EP 0 564 797 B1**
(45) Veröffentlichungstag und Bekanntmachung des Hinweises auf die Patenterteilung: **02.09.1998**
(21) Anmeldenummer: 93102936.7
(22) Anmeldetag: 25.02.1993
(51) Int. Cl.: A61M 5/158, A61M 25/06

(54) **Infusionsnadel**
Infusion needle
Aiguille d'infusion

(30) Priorität: 09.04.1992 DE 4211932
(43) Veröffentlichungstag der Anmeldung: 13.10.1993
(73) Patentinhaber: Willing, Erika, D-46397 Bocholt (DE)
(72) Erfinder: Willing, Erika, D-46397 Bocholt (DE)
(74) Vertreter: Cohausz & Florack Patentanwälte

(56) Entgegenhaltungen:
- WO-A-88/07388
- CH-A- 400 463
- FR-A- 2 245 383
- US-A- 4 518 383
- US-A- 4 846 805

## Beschreibung

Vorliegende Erfindung betrifft eine Infusionsnadel, insbesondere für die allgemeine Infusionstherapie, mit einem eine Metallkanüle und eine damit in Verbindung stehende zylinderförmige Aufnahmeöffnung zur Aufnahme von Applikatoren aufweisenden Nadelaufnahmekörper, wobei die Metallkanüle als Punktionskanüle ausgebildet ist und von einer etwas kürzeren, konisch endenden und dicht anliegenden sowie mit der Aufnahmeöffnung verbundenen Kunststoffkanüle umgeben ist.

Neben der oralen und rektalen Applikation von Medikamenten ist die parenterale Verabreichung von Pharmaka eine der Grundlagen der heutigen Therapie. Auch wenn in der modernen Medizin die intravenöse Infusionstherapie und arterielle Injektionstechnik die Injektionstherapie der drei klassischen Injektionsformen, also die subkutane, die intramuskuläre und die intravenöse Applikation von Medikamenten teilweise verdrängt hat, stellt die subkutane und intrakutane Applikation - insbesondere im Bereich der Schmerztherapie und der Behandlung von Diabetes - nach wie vor einen großen Bereich der Infusionstherapie dar. Ihre Entwicklung verlief in vielen kleinen Schritten und über einen langen Zeitraum. Methoden zum Aderlaß und zu Zwecken der Zuführung von Medikamenten sind schon lange Zeit bekannt.

Mit der Entdeckung des Blutkreislaufes zu Anfang des 17. Jahrhunderts war die anatomisch-physiologische Basis bezüglich der Infusion und Transfusion geschaffen worden. Schon bald begannen erste Infusionsversuche, erst bei Tieren und später auch am Menschen, die jedoch zunächst weder einen therapeutischen Effekt noch einen Fortschritt der Erkenntnis zu erzielen vermochten. Da der Grund für die anfänglichen Mißerfolge wohl in erster Linie auf die septischen Verhältnisse zurückzuführen war, ermöglichte erst die Erfindung der Injektionsspritze im 19. Jahrhundert den Weg zur modernen Injektions- und Infusionstherapie. Jedoch begann ein neues Kapitel in der Transfusionslehre erst nach der Blutgruppenentdeckung zu Anfang dieses Jahrhunderts. Erst damit konnten die entscheidenden Grundlagen und Techniken entwickelt werden, die - angeregt durch die beiden Weltkriege und die Entwicklung der inneren Medizin und der Anästhesiologie - größtenteils bis zum heutigen Tage noch Gültigkeit haben.

Die subkutane Injektion stellt im Bereich der Schmerztherapie immer noch einen wichtigen Bereich dar, obwohl die Häufigkeit therapeutischer, subkutaner Injektionen im Gegensatz zu früher deutlich zurückgegangen ist. Dieser Rückgang liegt zum einen daran, daß die Resorptionszeiten schlecht bestimmbar sind und daß sie stark von dem Zustand des jeweiligen Patienten abhängen. Zum anderen werden heute mehr differente Mittel injiziert, die - subkutan appliziert - unverträglich sind. Doch spielt die subkutane Technik und insbesondere die subkutanen Infusionen bei Kindern nach wie vor eine wesentliche Rolle.

Bei der subkutanen Infusion ist es sehr wichtig, daß die Injektionen lege artis durchgeführt werden, weil eine Injektion in die Kutis nicht nur starke Schmerzen verursacht, sondern zu heftigen Entzündungen bis hin zu Nekrosen der Haut führen kann. Ebenfalls bekannt sind die ödimatösen Quaddeln nach subkutaner Injektion, die mit mehr oder minder starker Entzündung einhergehen, so daß die Kranken oft recht empfindlich in ihrem Wohlbefinden beeinträchtigt sind. Jedoch sind diese Komplikationen meist harmlos und verschwinden in wenigen Tagen. In seltenen Fällen können sie aber auch zur Nekrose der Haut und durch Sekundärinfektion zu einem Erysipel und bei ungünstiger Ausgangslage des Patienten zu einer Sepsis führen. Die intrakutane Injektion spielt auch heute noch bei der BCG-Schutzimpfung (Bacille-Calmette- Guerin; Schutzimpfung gegen Tuberkulose) eine große Rolle. Sie wird vielfach in der Heiltherapie angewendet.

Bei der subkutanen Injektion bzw. Infusion muß das Medikament unter die Haut in das subkutane Fettgewebe gespritzt bzw. gegeben werden, während bei der intrakutanen Injektion ein Arzneimitteldepot direkt in der Haut (Kutis) zu liegen kommt. Die meisten subkutanen Injektionen werden im Bereich des Oberarms, des Oberschenkels oder der Brust verabreicht. Im Gegensatz zu den anderen Injektionsmethoden gibt es bei der subkutanen Injektionstechnik keinen wesentlichen Unterschied bei der Anwendung dieser Therapie zwischen Männern, Frauen, Kindern und Säuglingen.

Wärend bei der Injektion die Injektionsnadel nach dem Setzen eines Arzneimitteldepots durch Herausziehen wieder aus dem subkutanen oder intrakutanen Gewebe entfernt wird, verbleibt eine Infusionsnadel über einen längeren Zeitraum - gegebenenfalls bis zu einigen Tagen - am Applikationsort. Es ist schnell ersichtlich, daß dies für den Patienten unangenehm ist und ihn in seiner Bewegungsfreiheit behindert. Unumgängliche Bewegungen können mehr oder minder starke Schmerzen verursachen.

Aus der CH-PS 400 463 ist eine Kanüle für Infusionen, insbesondere für Dauertropfinfusionen mit einem eine Metallkanüle und eine damit in Verbindung stehende zylinderförmige Aufnahmeöffnung aufweisenden Nadelaufnahmekörper bekannt. Bei dieser bekannten Kanüle ist die Metallkanüle als Punktionskanüle ausgebildet und von einer etwas kürzeren, konisch endenden und dicht anliegenden sowie mit der Aufnahmeöffnung verbundenen Kunststoffkanüle umgeben. Diese bekannte Infusionsnadel weist eine Metallkanüle auf, welche in einem flachen Winkel zur Hautoberfläche im subkutanen oder intrakutanen Gewebe zu liegen kommt. Auch wenn die bekannte Infusionsnadel einfach auf der Haut zu befestigen ist, trägt sie jedoch - bedingt durch die Abwinklung - relativ hoch auf und hat demzufolge ein Scheuern z.B. auf der Kleidung des Patienten zur Folge, was wiederum mit Schmerzen für den Patienten verbunden sein kann.

Der vorliegenden Erfindung liegt daher die Aufgabe zugrunde, die bekannte und zuvor näher beschriebene Infusionsnadel so auszugestalten und weiterzubilden, daß eine bestmögliche Anpassung an Bewegungen des Patienten erreicht wird, ohne auf den sicheren Halt der Infusionsnadel am Applikationsort verzichten zu müssen.

Diese Aufgabe wird erfindungsgemäß dadurch gelöst, daß der Nadelaufnahmekörper im Übergangsbereich zwischen Aufnahmeöffnung und Kunststoffkanüle abgewinkelt ausgeführt ist, so daß die Aufnahmeöffnung des Nadelaufnahmekörpers nahezu parallel zur Hautoberfläche verläuft.

Erfindungsgemäß wird eine Infusionsnadel geschaffen, bei der die Metallkanüle nur noch für die eigentliche Injektion benötigt wird. Nach der Injektion der Infusionsnadel und deren Befestigung am Applikationsort wird die Metallkanüle, der sogenannte Mandrin - wie bei einer Braunüle (eingetragenes Warenzeichen der Firma B. Braun Melsungen AG) - aus der sie umschließenden Kunststoffkanüle entfernt. Relative Bewegungen der Subkutis zur Epidermis sind für den Patienten nunmehr nahezu schmerzfrei, da sich die flexible Kunststoffkanüle solchen Relativbewegungen anpassen kann, ohne ihre Position im wesentlichen zu verändern. Bedingt durch die Abwinklung der erfindungsgemäßen Infusionsnadel wird erreicht, daß die Aufnahmeöffnung des Nadelaufnahme - körpers nahezu parallel zur Hautoberfläche verläuft. Diese Abwinklung sollte zwischen 15 und 25° betragen. Als besonders vorteilhaft hat sich ein Winkel von 20° erwiesen.

Nach einer weiteren Lehre der Erfindung ist der Übergangsbereich zwischen der zylinderförmigen Aufnahmeöffnung und der Kunststoffkanüle konisch ausgebildet. Auf diese Weise wird erreicht, daß der Mandrin bei der Herstellung der Infusionsnadel beim Einführen in die Kunststoffkanüle diese nicht beschädigen kann und es wird darüber hinaus eine gleichmäßige Biegung des Mandrins im abgewinkelten Übergangsbereich zwischen Aufnahmeöffnung und Kunststoffkanüle ermöglicht.

In weiterer Ausgestaltung der Erfindung weist der Nadelaufnahmekörper im Bereich der Abwinklung eine flächige Verbreiterung auf, welche auf der die Kunststoffkanüle aufweisenden Seite mit einer elastischen Auflage versehen ist. Als besonders zweckmäßig hat sich als elastische Auflage ein entlang den Rändern der Verbreiterung verlaufender endloser Schaumstoffstreifen erwiesen. Eine solche elastische Auflage ist für sich bereits von den sog. "Gripper"-Nadeln, wie sie bei voll implantierbaren Kathetersystemen eingesetzt werden, bekannt.

Besonders zweckmäßig ist es, wenn die Metallkanüle an ihrem der Kanülenspitze abgewandten Ende ein Aufnahmeelement mit angeformter Griffplatte aufweist. Dadurch wird eine sichere Handhabung der erfindungsgemäßen Infusionsnadel beim Injizieren erreicht.

Eine weitere Lehre der Erfindung sieht vor, daß der Nadelaufnahmekörper im Bereich der Aufnahmeöffnung und/oder das Aufnahmeelement der Metallhülse Mittel aufweist, welche ein axiales Verdrehen der eingeführten Metallkanüle verhindern. Auf diese Weise wird - je nach Punktionsart - die gewünschte feste Zuordnung der schräg angeschliffenen Metallkanülenspitze zum Nadelaufnahmekörper gewährleistet.

Es gibt nun verschiedene Möglichkeiten, die Lehre der Erfindung auszugestalten und weiterzubilden, wozu einerseits auch die Unteransprüche und andererseits auch die nachfolgende Erläuterung eines bevorzugten Ausführungsbeispiels der erfindungsgemäßen Infusionsnadel anhand der Zeichnung verwiesen wird. In der Zeichnung zeigen
- Fig. 1: eine Seitenansicht der erfindungsgemäßen Infusionsnadel,
- Fig. 2: eine Draufsicht auf die erfindungsgemäße Injektionsnadel und
- Fig. 3: einen Längsschnitt durch die erfindungsgemäße Injektionsnadel entlang der Linie III-III aus Figur 2.

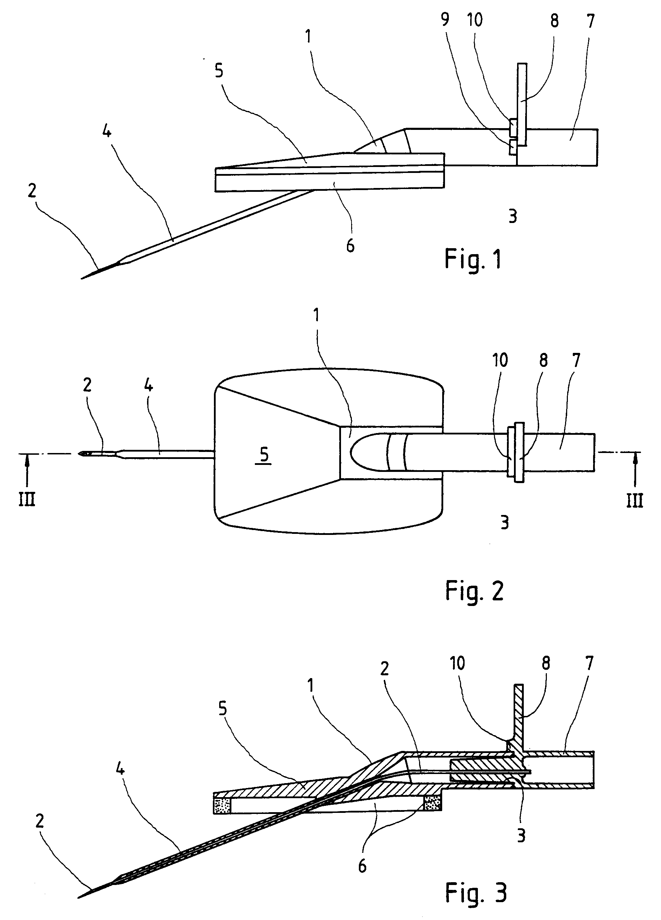

In Figur 1 und Figur 2 ist eine erfindungsgemäße Infusionsnadel mit einem Nadelaufnahmekörper 1 dargestellt, welcher zunächst eine Metallkanüle 2 und eine damit in Verbindung stehende zylinderförmige Aufnahmeöffnung 3 aufweist. Dabei ist die Metallkanüle 2 als Punktionsinnenkanüle ausgebildet und von einer etwas kürzeren, konisch endenden und dicht an ihr anliegenden sowie mit der Aufnahmeöffnung 3 verbundenen Kunststoffkanüle 4 umgeben.

Figur 3 zeigt die erfindungsgemäße Infusionsnadel nach Figur 1 im Längsschnitt. Aus ihr geht deutlich die Ausgestaltung der Aufnahmeöffnung 3 hervor. Die erfindungsgemäße Infusionsnadel zeichnet sich dadurch aus, daß die Metallkanüle 2 als Punktionskanüle ausgebildet ist und von einer etwas kürzeren, konisch endenden und dicht an ihr anliegenden sowie mit der Aufnahmeöffnung 3 verbundenen Kunststoffkanüle umgeben ist, daß der Nadelaufnahmekörper 1 im Übergangsbereich zwischen Aufnahmeöffnung 3 und Kunststoffkanüle 4 abgewinkelt ausgeführt ist, und daß die Metallkanüle 2 aus der sie umschließenden Kunststoffkanüle 4 und dem Nadelaufnahmekörper 1 herausziehbar ausgebildet ist. Wie bereits erwähnt, ist es vorteilhaft, wenn die Abwinklung zwischen 15 und 25° beträgt. Im dargestellten und insoweit bevorzugten Ausführungsbeispiel ist ein Winkel von 20° vorgesehen.

Aus Figur 3 ist ferner zu entnehmen, daß der Übergangsbereich zwischen der zylinderförmigen Aufnahmeöffnung 3 und der Kunststoffkanüle 4 konisch ausgebildet ist. Hierdurch wird zum einen das Einführen der Metallkanüle 2 und die Kunststoffkanüle 4 erleichtert und zum anderen erhält die Metallkanüle 2 einen ausreichenden Freiraum für ihre durch die Abwinklung erforderliche Biegung.

Zur Befestigung der erfindungsgemäßen Infusionsnadel am Applikationsort weist der Nadelaufnahmekörper 1 im Bereich der Abwinklung eine flächige Verbreiterung 5 auf. Wie insbesondere aus den Figuren 1 und 3 hervorgeht, ist diese Verbreiterung 5 auf der die Kunststoffkanüle 4 aufweisenden Unterseite des Nadelaufnahmekörpers 1 mit einer elastischen Auflage versehen, welche vorzugsweise als ein entlang den Rändern der Verbreiterung 5 verlaufender endloser Schaumstoffstreifen 6 vorgesehen ist.

Die Figuren zeigen ferner, daß die Metallkanüle 2 an ihrem der Kanülenspitze abgewandten Ende ein Aufnahmeelement 7 mit angeformter Griffplatte 8 aufweist.

Auf diese Weise ist es möglich, auch durch die gesetzte Metallkanüle 2 bereits ein Medikament zu injizieren.

Zur besseren Handhabung ist schließlich vorgesehen, daß der Nadelaufnahmekörper 1 im Bereich der Aufnahmeöffnung 3 und das Aufnahmeelement 7 der Metallkanüle 2 Mittel aufweisen, die ein axiales Verdrehen der eingeführten Metallkanüle 2 verhindern. Hierzu weist der Nadelaufnahmekörper 1, wie aus Figur 1 hervorgeht, seitliche Vorsprünge 9 und das Aufnahmeelement 7 einen ringsegmentartigen Steg 10 auf. Dies ist für eine sichere Handhabung unumgänglich, da die Metallkanüle 2 im Bereich ihrer Spitze unter einem flachen Winkel angeschrägt ausgeführt ist, um ein leichteres Eindringen in die Haut zu gewährleisten. Nach dem Setzen der erfindungsgemäßen Infusionsnadel ruht der Nadelaufnahmekörper 1 mit seiner mit der elastischen Auflage 6 versehenen Verbreiterung 5 auf der Oberfläche der Haut des Patienten. Nachdem dort eine entsprechende Fixierung stattgefunden hat, läßt sich der Mandrin 2 aus der Kunststoffkanüle 4 herausziehen und ein entsprechender Applikator kann mit Aufnahmeöffnung 3 des Nadelaufnahmekörpers 1 verbunden werden.

Aus den Figuren geht deutlich hervor, daß nach herausgezogenem Mandrin 2 einschließlich Aufnahmeelement 7 und Griffplatte 8 der gesamte Nadelaufnahmekörper 1 nur noch sehr gering aufträgt, nämlich im wesentlichen nur noch um die Höhe der Außenabmessung des Nadelaufnahme körpers 1 im Bereich der Aufnahmeöffnung 3, da der Schaumstoffstreifen 6 durch seine Fixierung am Applikationsort noch zusammengedrückt wird.

## Patentansprüche

1. Infusionsnadel, insbesondere für die allgemeine Infusionstherapie, mit einem eine Metallkanüle und eine damit in Verbindung stehende zylinderförmige Aufnahmeöffnung aufweisenden Nadelaufnahmekörper (1), wobei die Metallkanüle als Funktionskanüle ausgebildet ist, von einer etwas kürzeren, konisch endenden und dicht anliegenden sowie mit der Aufnahmeöffnung (3) verbundenen Kunststoffkanüle (4) umgeben und aus dieser und dem Nadelaufnahmekörper (1) herauziehbar ausgebildet ist, **dadurch gekennzeichnet**, daß der Nadelaufnahmekörper (1) im Übergangsbereich zwischen Aufnahmeöffnung (3) und Kunststoffkanüle (4) abgewinkelt ausgeführt ist, so daß die Aufnahmeöffnung (3) des Nadelaufnahmekörpers (1) nahezu parallel zur Hautoberfläche verläuft.

2. Infusionsnadel nach Anspruch 1, **dadurch gekennzeichnet**, daß die Abwinklung zwischen 15 und 25°, vorzugsweise 20° beträgt.

3. Infusionsnadel nach Anspruch 1 oder 2, **dadurch gekennzeichnet,** daß der Übergangsbereich zwischen der zylinderförmigen Aufnahmeöffnung (3) und der Kunststoffkanüle (4) konisch ausgebildet ist.

4. Infusionsnadel nach einem der Ansprüche 1 bis 3, **dadurch gekennzeichnet,** daß der Nadelaufnahmekörper (1) im Bereich der Abwinklung eine flächige Verbreiterung (5) aufweist und daß die Verbreiterung (5) auf der die Kunststoffkanüle (4) aufweisenden Seite mit einer elastischen Auflage versehen ist.

5. Infusionsnadel nach Anspruch 4, **dadurch gekennzeichnet,** daß als elastische Auflage ein entlang den Rändern der Verbreiterung (5) verlaufender endloser Schaumstoffstreifen (6) vorgesehen ist.

6. Infusionsnadel nach einem der Ansprüche 1 bis 5, **dadurch gekennzeichnet,** daß die Metallkanüle (2) an ihrem der Kanülenspitze abgewandten Ende ein Aufnahmeelement (7) mit angeformter Griffplatte (8) aufweist.

7. Infusionsnadel nach Anspruche 6, **dadurch gekennzeichnet,** daß der Nadelaufnahmekörper (1) im Bereich der Aufnahmeöffnung (3) und/oder das Aufnahmeelement (7) der Metallkanüle (2) Mittel (9,10) aufweisen bzw. aufweist, welche ein axiales Verdrehen der eingeführten Metallkanüle (2) verhindern.

## Claims

1. An infusion needle, more particularly for general infusion therapy, having a metal cannula and a needle receiving member (1) having a cylindrical receiving opening and connected to the metal cannula, the metal cannula taking the form of a puncture cannula enclosed tightly by a somewhat shorter, conically terminating plastics cannula (4) which is connected to the receiving opening (3) and from which and from the needle receiving member (1) the metal cannula can be withdrawn, characterised in that in the transitional zone between the receiving opening (3) and the plastics cannula (4) the needle receiving member (1) is bent at an angle, so that the receiving opening (3) of the needle receiving member (1) extends substantially parallel with the surface of the skin.

2. An infusion needle according to claim 1, characterised in that the bending angle is between 15 and 25°, preferably 20°.

3. An infusion needle according to claim 1 or 2, characterised in that the transitional zone between the cylindrical receiving opening (3) and the plastics cannula (4) is constructed conically.

4. An infusion needle according to claims 1 to 3, characterised in that in the zone of the bend the needle receiving member (1) has a flat widened portion (5), the widened portion (5) being provided with a resilient support on the side having the plastics cannula (4).

5. An infusion needle according to claim 4, characterised in that the resilient support provided is an endless strip of cellular material (6) extending along the edges of the widened portion (5).

6. An infusion needle according to claims 1 to 5, characterised in that at its end remote from its tip the metal cannula (2) has a receiving element (7) having a moulded-on gripping plate (8).

7. An infusion needle according to claim 6, characterised in that the needle receiving member (1) has in the zone of the receiving opening (3) and/or the receiving element (7) for the metal cannula (1) has means (9, 10) which prevent any axial twisting of the introduced metal cannula (2).

## Revendications

1. Aiguille de perfusion, notamment pour une thérapie générale de perfusion, comprenant un corps (1) de réception d'aiguille présentant une canule métallique et une ouverture de réception cylindrique qui est en liaison avec la canule métallique, la canule métallique étant réalisée sous la forme d'une canule fonctionnelle entourée d'une canule (4) en matière plastique qui est un peu plus courte que la canule fonctionnelle et se termine de façon conique, et qui est en contact étroit avec la canule fonctionnelle et est reliée à l'ouverture de réception (3), la canule fonctionnelle étant réalisée de sorte qu'elle puisse être retirée de la canule en matière plastique et du corps (1) de réception de l'aiguille, caractérisée en ce que le corps de réception de l'aiguille (1) est réalisé de façon coudée dans la zone de transition entre l'ouverture de réception (3) et la canule en matière plastique (4) de sorte que l'ouverture de réception (3) du corps (1) de réception de l'aiguille s'étend de façon pratiquement parallèle à la surface de la peau.

2. Aiguille de perfusion selon la revendication 1, caractérisée en ce que la partie coudée fait un angle compris entre 15 et 25°, et de préférence de 20°.

3. Aiguille de perfusion selon la revendication 1 ou 2, caractérisée en ce que la zone de transition entre l'ouverture de réception cylindrique (3) et la canule en matière plastique (4) est réalisée de façon conique.

4. Aiguille de perfusion selon l'une quelconque des revendications 1 à 3, caractérisée en ce que le corps de réception de l'aiguille (1) présente, dans la zone de coudage, un élargissement surfacique (5) et en ce que l'élargissement (5) est muni d'un appui élastique sur la face présentant la canule en matière plastique (4).

5. Aiguille de perfusion selon la revendication 4, caractérisée en ce qu'en tant qu'appui élastique, il est prévu une bande (6) de mousse sans fin s'étendant le long des bords de l'élargissement (5).

6. Aiguille de perfusion selon l'une quelconque des revendications 1 à 5, caractérisée en ce que la canule métallique (2) présente à son extrémité opposée à la pointe de la canule un élément de réception (7) avec une plaque de poignée (8) venue de matière.

7. Aiguille de perfusion selon la revendication 6, caractérisée en ce que le corps de réception de l'aiguille présente dans la zone de l'ouverture de réception (3) et/ou de l'élément de réception (7) de la canule métallique (2) un moyen (9, 10) qui empêche une rotation axiale de la canule métallique (2) introduite.
